# EUROPEAN PATENT APPLICATION

(11) **EP 3 845 214 A1**
(43) Date of publication of application: **07.07.2021**
(21) Application number: 19856271.2
(22) Date of filing: 20.08.2019
(51) Int. Cl.: A61J 3/00, B65B 1/30

(54) **MEDICINE LOADING DEVICE AND MEDICINE RECOVERY METHOD**

(30) Priority: 28.08.2018 JP 2018159036
(71) Applicant: YUYAMA MFG. CO., LTD., Toyonaka-shi, Osaka 561-0841 (JP)
(72) Inventor: YUYAMA, Hiroyuki, Toyonaka-shi, Osaka 561-0841 (JP)
(74) Representative: AOMB Polska Sp. z.o.o.
(86) International application number: PCT/JP2019/032465
(87) International publication number: WO 2020/045169

(57) **Abstract**

The present invention provides a medicine loading device which can prevent an improper medicine from being loaded based on prescription information. The medicine loading device has a main body, a medicine dispensing cassette and medicine discriminating means. An openable/closable lid and a locking mechanism for locking the cover are included in the medicine dispensing cassette. When a type of a medicine detected by the medicine discriminating means matches a type of a medicine to be contained in the medicine dispensing cassette, the locking mechanism is released.

## Description

### TECHNICAL FIELD

The present invention relates to a medicine loading device for loading a medicine based on prescription information, and a medicine recovery method.

### BACKGROUND ART

There is generally known medicine dispensing devices which automatically dispense a prescribed medicine based on a prescription. For example, the applicant of the present application has developed a medicine dispensing device described in patent document 1, and this medicine dispensing device has gained a good reputation and has obtained high evaluation that efficiencies of prescription operations have been increased from prescription operators.

In a conventional medicine dispensing device, various medicines to be dispensed are stored in a plurality of cassettes. When the medicines have been dispensed and the medicines in the cassette has been lack of short, the prescription operator needs to remove the cassette from the device, discriminates medicines and refills them into the medicine cassette with his/her own hands.

On the other hand, although it is preferable that an amount of the prescribed medicines and a dosage of the prescribed medicines should match a required amount depending on a treatment content for a patient, in general, it is often found that there are unused medicines and time-expired medicines due to forgetfulness of taking medicines of the patient or a change in a medical treatment content. Since annual unused medicines and time-expired medicines are generally in enormous quantities, if these unused or time-expired medicines are wasted as they are, it would be significant wasteful and thus it is required to appropriately recover these medicines.

However, if a package or identification information for the unused medicines or time-expired medicines breaks, there is often a case that the medicines are brought in an unpacked state, for example. In this case, the prescription operator is forced to discriminate the type of the medicines by his/her own eyes from only appearance of the medicines, and at this time, a mistake that an improper medicine is refilled into the cassette may occur.

One of objects of the present invention is to prevent the improper medicine from being refilled into the medicine dispensing cassette.

### PRIOR ART DOCUMENT

### PATENT DOCUMENT

Patent document 1: WO 2010/110360

### SUMMARY OF THE INVENTION

### PROBLEM TO BE SOLVED BY THE INVENTION

The present invention provides a medicine loading device which can prevent an improper medicine from being loaded based on prescription information.

### MEANS FOR SOLVING THE PROBLEM

A first aspect of the present invention relates to a medicine loading device for loading a medicine based on prescription information. This medicine loading device has a main body, a medicine dispensing cassette and medicine discriminating means. The at least one medicine dispensing cassette is provided in the main body. An openable/closable lid is provided at the medicine dispensing cassette. Further, a locking mechanism for locking the lid is provided at the medicine dispensing cassette. When a type of a medicine detected by the medicine discriminating means and a type of a medicine to be contained in the medicine dispensing cassette match each other, the locking mechanism is released.

It is preferable that the medicine discriminating means further has a medicine information obtaining part for reading cassette information provided at either one of the medicine dispensing cassette and the lid and medicine information provided on the medicine and an information processing part connected to the medicine information obtaining part. The information processing part preferably discriminates the type of the medicine based on information on the medicine obtained by the medicine information obtaining part.

It is preferable that the medicine information obtaining part has a photographing device for obtaining an image in which the information on the medicine which is a discrimination target is indicated and at least one of a first reading device which can read a code in which the information on the medicine which is the discrimination target is indicated and a second reading device which can read a signal indicating the medicine information of the discrimination target.

It is preferable that the image in which the medicine which is the discrimination target is indicated is an image of an engraved mark or a print image of the medicine, and the code in which the medicine which is the discrimination target is indicated is any one of a bar code, a two-dimensional code and a three-dimensional code indicating the medicine information on the medicine.

It is preferable that the locking mechanism further has a trigger for allowing a lock to be released. This trigger can further return the locking mechanism to a locked state after releasing the locking mechanism. The trigger more preferably allows the cassette to be removed from the medicine loading device and the trigger is returned from a releasing state of the lock to an original state. Further, it is more preferable that the trigger has a receiving part for receiving an instruction for allowing the trigger to return from the releasing state of the lock to the original state from an outside after the cassette has completed a loading operation. Further, it is preferable that the trigger further has a timer part and the trigger is returned from the releasing state of the lock to the original state when a predetermined time elapses after the locking mechanism has been released by this timer part.

It is preferable that the medicine loading device further has a storage part which can store predetermined medicine information in advance and further store the information on the medicine detected by the medicine discriminating means, and this storage part stores an image of the medicine, a numerical value or a rank indicating a degree of possibility of correctness and the engraved mark or a print code of the medicine.

It is preferable that the medicine loading device further has a control part which creates template image data based on the stored information on the medicine and performs pattern matching for calculating a score serving as an index of a degree of matching between image data of the engraved mark and/or a print of the medicine and the template image data to discriminate the medicine.

It is preferable that the medicine loading device further has a display and this display displays a result of comparing the information on the medicine detected by the medicine discriminating means with the information on the medicine stored in the storage part.

It is preferable that the medicine loading device further has an alert part for alerting an operator when the type of the medicine detected by the medicine discriminating means and the type of the medicine to be contained in the medicine dispensing cassette do not match each other.

A second aspect of the present invention relates to a medicine recovery method for recovering a medicine based on prescription information. This medicine recover method has a step of obtaining medicine information indicating a type of a medicine which is a discrimination target, a step of comparing the medicine information with cassette information indicating a type of a medicine to be contained in a medicine dispensing cassette, a step of determining whether the medicine information and the cassette information match each other, and a step of releasing a locking mechanism provided at the medicine dispensing cassette when the medicine information and the cassette information match each other.

A third aspect of the present invention relates to a medicine loading support device for preventing an improper medicine from being loaded into medicine holding means. This medicine loading device has a main body, at least one medicine holding means contained in the main body, an opening and closing part attached to the medicine holding means, a locking mechanism for locking the opening and closing part, and discriminating means for discriminating a type of a medicine. When the type of the medicine discriminated by the discriminating means and a type of a medicine to be contained in the medicine holding means match each other, the locking mechanism is released.

### EFFECTS OF THE INVENTION

According to the present invention, it is possible to prevent the improper medicine from being refilled into the medicine dispensing cassette.

### BRIEF DESCRIPTION OF THE DRAWINGS

[Fig. 1] Fig. 1 is a perspective view showing an embodiment of a medicine dispensing device.
[Fig. 2] Fig. 2 is a perspective view of a medicine dispensing cassette included in the medicine dispensing device. In this regard, this figure is a view of the medicine dispensing cassette seen from the front right side.
[Fig. 3] Fig. 3 is a perspective view of the medicine dispensing cassette. In this regard, this figure is a view of the medicine dispensing cassette viewed from the rear right side. Figs. 4-9 are mainly used to explain a locking mechanism included in the medicine dispensing cassette.
[Fig. 4] Fig. 4 is a bottom view of the medicine dispensing cassette.
[Fig. 5] Fig. 5(a) is a perspective view of a lid included in the medicine dispensing cassette. In this regard, this figure is a view of the lid viewed from the lower rear side. Fig. 5(b) is a cross-sectional rear view of the lid. In this regard, this figure is a view of the lid viewed from the rear side toward the front side.
[Fig. 6] Fig. 6 is a bottom view of members constituting a medicine dispensing cassette main body.
[Fig. 7] Fig. 7 is a perspective view of the locking mechanism included in the medicine dispensing cassette main body.
[Fig. 8] Fig. 8 is a cross-sectional rear view of the vicinity of the locking mechanism in the medicine dispensing cassette. In this regard, this figure is a view of the locking mechanism viewed from the rear side toward the front side of the medicine dispensing cassette.
[Fig. 9] Fig. 9 is a schematic diagram illustrating a mechanism for enabling the locking mechanism to lock/unlock the lid. Figs. 10 to 16 mainly show a configuration of a medicine photographing device.
[Fig. 10] Fig. 10 is a perspective view of an embodiment of the medicine photographing device.
[Fig. 11] Fig. 11 is a perspective view showing a state that a front cover of the medicine photographing device shown in Fig. 10 is opened.
[Fig. 12] Fig. 12 is a perspective view showing an internal configuration of the medicine photographing device shown in Fig. 10. This figure mainly shows a frame and members attached to the frame.
[Fig. 13] Fig. 13 is a front view of the medicine photographing device shown in Fig. 12.
[Fig. 14] Fig. 14(a) is a perspective view showing an overall shape of a support member. Fig. 14(b) is a side view showing a positional relationship among a first light source, a second light source and a medicine placing item.
[Fig. 15] Fig. 15(a) is a perspective view showing an embodiment of a tray set in the medicine photographing device. Fig. 15(b) is a top view of the tray. Fig. 15(c) is a side view of the tray.

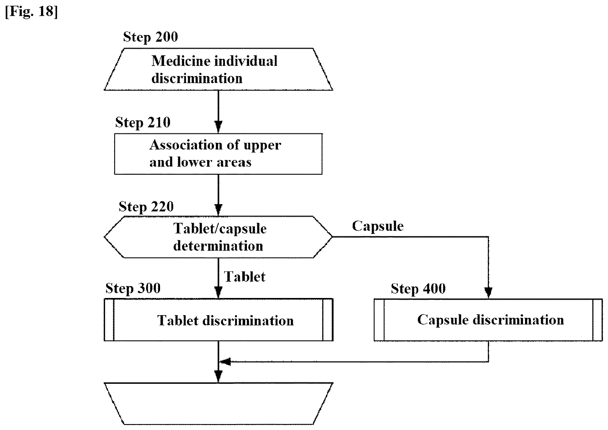

[Fig. 16] Fig. 16 is a perspective view of a petri dish attached to the tray shown in Fig. 15. Figs. 17 to 21 show a processing procedure for performing automated discrimination for the medicine.
[Fig. 17] Fig. 17 is a flowchart showing processes at an upstream portion of the discrimination.
[Fig.18] Fig.18 is a flowchart showing processes of medicine individual discrimination.
[Fig. 19] Fig. 19 is a flowchart illustrating processes of tablet discrimination.
[Fig. 20] Fig. 20 is a flowchart illustrating processes of capsule discrimination.
[Fig. 21] Fig. 21(a) is a perspective view of a calibration sheet used for calibration of a camera. Fig. 21(b) is a planar view of the calibration sheet.

### DETAILED DESCRIPTION OF THE INVENTION

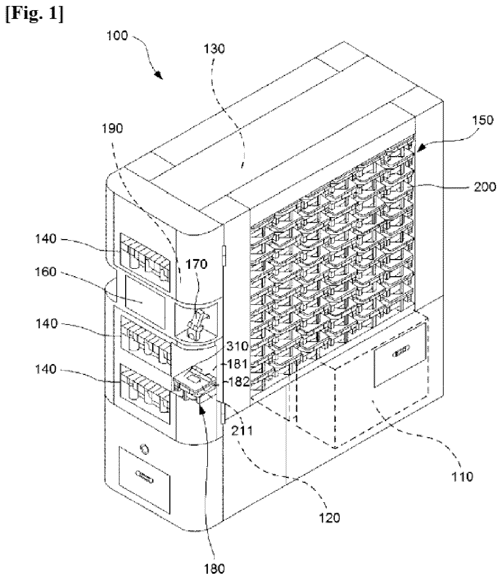

§1 Summary of medicine loading device Fig. 1 is a perspective view showing an embodiment of a medicine dispensing device. The medicine dispensing device 100 shown in this figure can dispense a prescribed medicine into a vial bottle based on input prescription information. This medicine dispensing device 100 has a vial bottle feeding device 110, a label attachment device 120, a vial bottle carrying device 130 and discharge ports 140. Further, the medicine dispensing device 100 has cassette arranged portions 150 on side surfaces thereof. In addition, the medicine dispensing device 100 has a touch panel display 160, a scanner 170 and a medicine refilling portion 180 on a front surface thereof. Further, the medicine dispensing device 100 has a control device 190 therein. In addition, it has an alert part (not shown) for alerting a user when a type of a medicine detected by medicine discriminating means and a type of a medicine to be contained in a medicine dispensing cassette do not match each other.

Details of this medicine dispensing device 100 have been disclosed in WO 2010/110360. Further, details of a mechanism for enabling a cassette attached to the cassette arranged portion 150 to dispense the medicine have been disclosed in WO 2013/035692. Thus, in the following description, an overview of the medicine dispensing device 100 will be briefly described.

As shown in Fig. 1, the vial bottle feeding device 110 is provided on the inner lower side and the rear side of the medicine dispensing device 100. This vial bottle feeding device 110 also has a function of storing a plurality of vial bottles. The label attachment device 120 is provided on the inner lower side and the front side of the medicine dispensing device 100. The cassette arranged portions 150 are provided on both side surfaces of an upper side of the medicine dispensing device 100. A plurality of medicine dispensing cassettes 200 (each of which is also called as a container) are arranged in these cassette arranged portions 150 in a matrix. The medicines to be dispensed by the medicine dispensing device 100 are stored in these medicine dispensing cassettes 200. The vial bottle carrying device 130 is provided between the cassette arranged portion 150 and the cassette arranged portion 150, that is, provided on the inner upper side of the medicine dispensing device 100. Further, the plurality of discharge ports 140 (three discharge ports 140 in the example shown in Fig. 1) are provided on the front surface of the medicine dispensing device 100.

The touch panel display 160 has both a function as a display device and a function as an input device. In addition, it can display a result of comparing information on the medicine detected by the medicine discriminating means with information on the medicine stored in a storage part. The scanner 170 can read a scanner reading symbol such as an image, a bar code, a two-dimensional code and an RF signal in which the information on the medicine is indicated. The control device 190 controls operations of various devices included in the medicine dispensing device 100 based on input information from the touch panel display 160, the scanner 170 and the like.

When the medicine dispensing device 100 dispenses the medicine, the scanner 170 first scans the symbol attached to a prescription to obtain prescription information. When the medicine dispensing device 100 receives an input of the prescription information and receives an input of a medicine dispensing instruction from a prescription operator, the vial bottle feeding device 110 first feeds a vial bottle to the label attachment device 120. Next, the label attachment device 120 prints a label based on the input prescription information and attaches this label to the vial bottle. Thereafter, the vial bottle carrying device 130 carries the vial bottle to which the label is attached into the vicinity of the medicine dispensing cassette 200 in which the prescribed medicines are stored. Then, the medicine dispensing cassette 200 dispenses the prescribed medicines into the vial bottle by a prescribed number. When the prescribed medicines are loaded in the vial bottle, the vial bottle carrying device 130 carries the vial bottle to the discharge port 140. Then, the prescription operator such as a pharmacist or a technician takes out the vial bottle discharged from the discharge port 140.

While the medicine dispensing device 100 is dispensing medicines, one of the medicine dispensing cassettes 200 is emptied in due course of time. In that case, the prescription operator needs to refill new medicines into this medicine dispensing cassette 200. However, in the medicine dispensing cassette 200, a lid 310 of the cassette is locked so that the lid 310 cannot be opened in a normal state. Therefore, at the time of refilling the medicines, the prescription operator needs to perform a predetermined operation for releasing the lock of the lid 310.

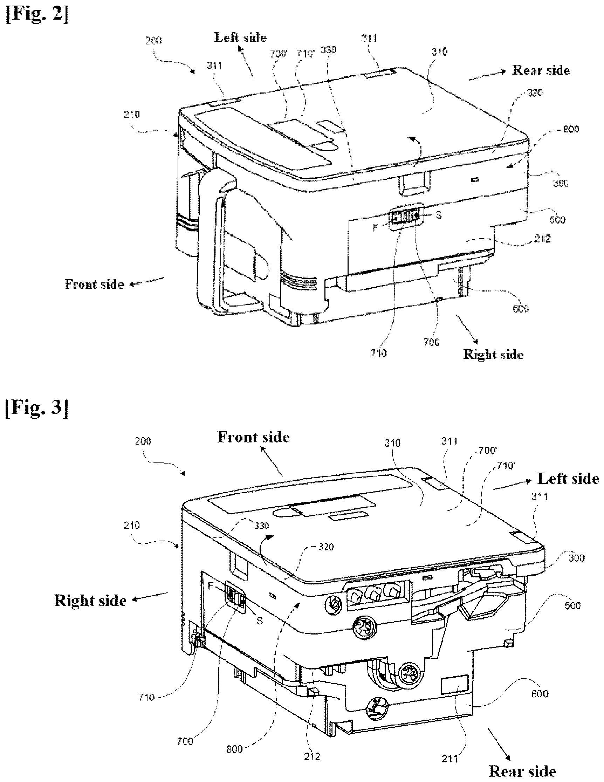

First, the prescription operator places the medicine dispensing cassette 200 to be refilled onto the medicine refilling portion 180. As shown in Fig. 3, an RF tag 211 is provided on a main body 210 of the medicine dispensing cassette 200 as an identification label for identifying the medicine dispensing cassette 200. Referring back to Fig. 1, the medicine dispensing device 100 includes an RF tag reading device 181 at the medicine refilling portion 180. Therefore, when the medicine dispensing cassette 200 is placed onto the medicine refilling portion 180, the medicine dispensing device 100 can identify the medicine dispensing cassette 200 placed on the medicine refilling portion 180 through the RF tag reading device 181. Then, the medicine dispensing device 100 holds information on the medicines stored in each of the medicine dispensing cassettes 200 therein. Therefore, based on this information, the medicine dispensing device 100 can identify the medicines to be refilled into the medicine dispensing cassette 200 placed on the medicine refilling portion 180.

At the time of refilling the medicine, the prescription operator makes the scanner 170 read the medicine to identify the type of the medicine to be refilled, which has been recovered from a warehouse or a patient. With this configuration, the medicine dispensing device 100 can identify the medicine to be refilled. The medicine dispensing device 100 has an unlocking device 182 for releasing the lock of the lid 310 of the medicine dispensing cassette 200 at the medicine refilling portion 180. Only when the medicine whose type has been identified is proper as the medicine to be refilled into the medicine dispensing cassette 200, the medicine dispensing device 100 drives the unlocking device 182 to release the lock of the lid 310 of the medicine dispensing cassette 200. With this configuration, the prescription operator can open the lid 310 and refill the new medicine into the medicine dispensing cassette 200. The medicine dispensing cassette 200 of the present embodiment has one feature regarding a locking mechanism for locking the lid 310. Hereinafter, the medicine dispensing cassette 200 including this locking mechanism will be described in detail.

§2 Summary of the medicine dispensing cassette 200 Figs. 2 and 3 are perspective views of the medicine dispensing cassette 200. In this regard, Fig. 2 is a view of the medicine dispensing cassette 200 viewed from the front right side and Fig. 3 is a view of the medicine dispensing cassette 200 viewed from the rear right side. In this specification, for convenience of description, a surface of the medicine dispensing cassette 200 facing the outside of the medicine dispensing device 100 is referred to as a "front surface" and a surface of the medicine dispensing cassette 200 facing the inside of the medicine dispensing device 100 is referred to as a "rear surface" when the medicine dispensing cassette 200 is set in the medicine dispensing device 100. Further, a portion of the medicine dispensing cassette 200 located on the right side is referred to as a "right side" and a portion of the medicine dispensing cassette 200 located on the left side is referred to as a "left side" when the medicine dispensing cassette 200 is viewed from the front side.

As shown in Figs. 2 and 3, in the medicine dispensing cassette 200, the lid 310 is attached to an upper surface of the main body 210. The lid 310 is hinged to the main body 210 at its left end by a hinge 311. Therefore, when the lock of the lid 310 is released, the lid 310 pivots around the hinge 311 to open. In other words, when the lock of the lid 310 is released, a right end of the lid 310 rises up and the lid 310 opens.

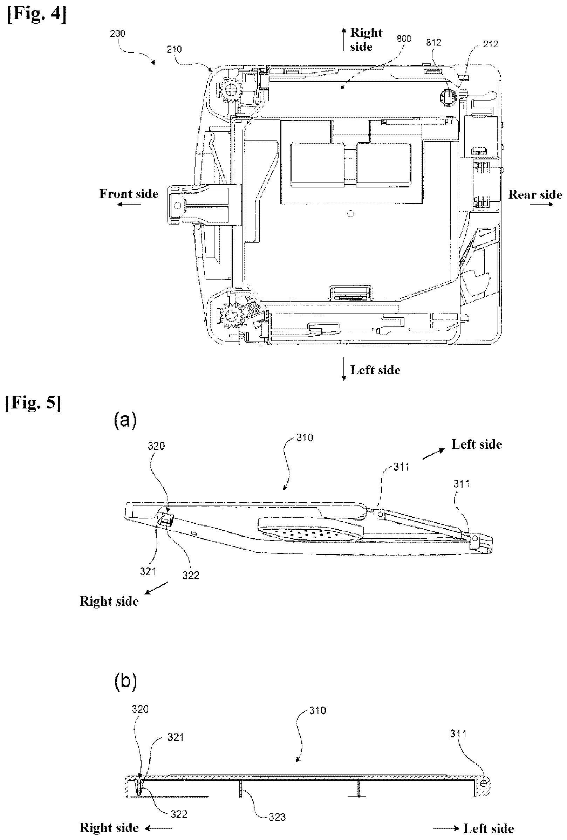

The medicine dispensing cassette 200 has a locking mechanism 800 for locking the lid 310 therein. Fig. 4 is a bottom view of the medicine dispensing cassette 200. Namely, Fig. 4 is a view of the medicine dispensing cassette 200 viewed from the lower side toward the upper side. As shown in this figure, a hole 212 is provided on a bottom surface of the main body 210. More specifically, the hole 212 is formed in the right side of the bottom surface of the main body 210 and in the vicinity of the rear surface of the main body 210 and opened in the vertical direction. The unlocking device 182 (see Fig. 1) of the medicine dispensing device 100 includes a rod (not shown) and this rod is configured to be inserted through the hole 212. Further, the unlocking device 182 operates the locking mechanism 800 with this rod to release the lock of the lid 310. If the hole 212 is provided in the bottom surface of the main body 210 as described in the present embodiment, assembly of the unlocking device 182 becomes easier. In addition, the user cannot easily release the lock of the locking mechanism 800.

§2.1 Structure of the lid 310 Fig. 5(a) is a perspective view of the lid 310 viewed from the lower side and Fig. 5(b) is a longitudinal cross-sectional view of the lid 310. In this regard, note that Fig. 5(b) shows the lid 310 viewed from the rear side toward the front side. As shown in these figures, an engaging portion 320 is provided in the vicinity of the right end of the lid 310, that is, in the vicinity of the end opposite to the hinge 311. The engaging portion 320 engages with the locking mechanism 800. The engaging portion 320 is constituted of a protruding portion 321 downwardly protruding from a bottom surface of the lid 310 and a slit 322 formed on the protruding portion 321. This slit 322 extends in the front-rear direction and its opening is opened toward the rear direction. A plurality of ribs 323 are provided between both ends so as to be parallel to each other.

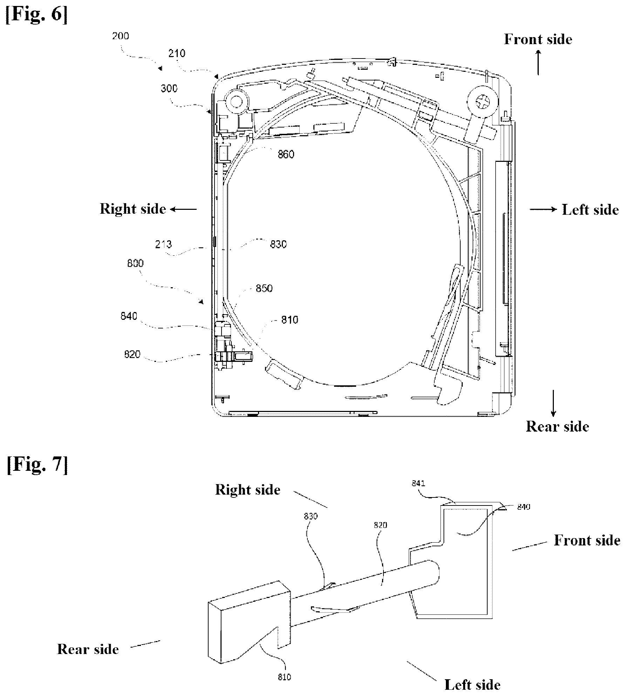

§2.2 Structure of the locking mechanism 800 Fig. 6 is a view of the inside of the main body 210 viewed from the lower side toward the upper side. Strictly speaking, as described later, the main body 210 is constituted of a main member 500, a first sub-member 300 and a third sub-member 600 as shown in Fig. 2 and Fig. 6 is a view of the first sub-member 300 viewed from the lower side toward the upper side in a state that a bottom cover of the first sub-member 300 is removed. As shown in this figure, the locking mechanism 800 is provided in the main body 210. More specifically, the locking mechanism 800 is provided at an upper portion of the main body 210 and in the vicinity of a right side surface of the main body 210.

The locking mechanism 800 further has a trigger for allowing the lock to be released and can use this trigger to release the locking mechanism 800 and then again return it to a locked state. Specifically, it can allow the cassette to be removed from the medicine loading device and return the trigger from a releasing state of the lock to an original state and further it can return to the locked state by receiving an instruction for returning the trigger from the releasing state of the lock to the original state from the outside after the cassette has completed the loading operation. Further, it is to return the trigger from the releasing state of the lock to the original state when a predetermined time elapses after the locking mechanism has been released.

Fig. 7 is a perspective view showing the locking mechanism 800. As shown in this figure, the locking mechanism 800 has an inclined portion 810, a shaft 820 and an engaging member 840 and further has a biasing member 830 as one example of the trigger. The engaging member 840 is attached to the vicinity of a front end of the shaft 820. The inclined portion 810, the shaft 820, the biasing member 830 and the engaging member 840 are integrally formed of one material (for example, resin, plastic, metal, rubber). This facilitates manufacture of the locking mechanism 800 and assembly of the medicine dispensing cassette 200. The biasing member 830 is attached to the vicinity of a rear end of the shaft 830.

As shown in Fig. 6, the shaft 820 is provided in the vicinity of a right inner wall 213 of the main body 210 so as to face toward the front-rear direction and be parallel to 310. Therefore, the shaft 820 faces toward a direction perpendicular to a central axis of the hole 212 (see Fig. 4). As shown in Fig. 7, the engaging member 840 is provided so as to face toward the upper side in an initial state (an unoperated state). A pawl 841 is formed at a tip end portion of the engaging member 840 so as to face the right inner wall 213 of the main body 210. Further, the biasing member 830 is formed in a tripod shape at a central portion of the shaft 820 and this tripod portion biases the shaft 820 toward the front direction with resistance force between the ribs.

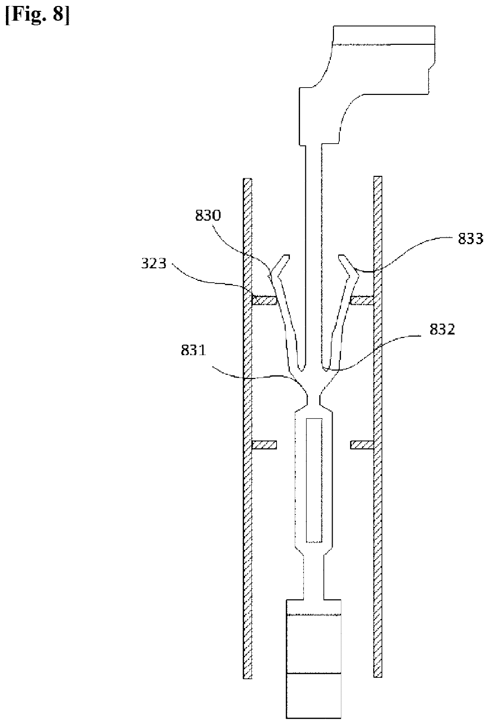

As shown in Fig. 8, both ends of the biasing member 830 are made of a substantially V or U-shaped plate-like material, that is a leaf spring having a shape such as a so-called triple crotch. Therefore, the biasing member 830 has a base end portion 831, a curved portion 832 and a tip end portion 833. The base end portion 841 is provided at the central portion of the shaft 830. The biasing member 830 is curved with an acute angle at the curved portion 832. Therefore, the tip end portion 833 extends straight at both oblique ends. The tip end portion 833 contacts with the rib 322 of the lid 310.

An inclined surface of the inclined portion 810 is configured to contact with a protruding bar portion and is located vertically above the hole 212 as shown in Fig. 4. As shown in Fig. 7, this inclined portion 810 faces in a direction perpendicular to the central axis of the hole 212 in the initial state.

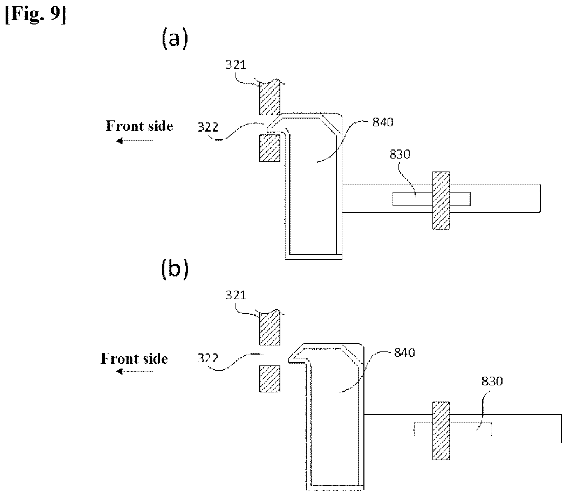

Fig. 9 is a schematic diagram illustrating a mechanism for enabling the locking mechanism 800 to lock/unlock the lid 310. As shown in Fig. 9(a), the engaging member 840 protrudes so as to face the front side due to biasing force of the biasing member 830 in the initial state. Further, in this state, the protruding engaging member 840 is fitted into the slit 322 formed in the lid 310 (see Fig. 5) and engaged with it.

As described above, the unlocking device 182 of the medicine dispensing device 100 can push the inclined portion 810 through the hole 212 (see Fig. 4) to move the entire portion of the shaft 820 in the rear direction. As a result, the shaft 820 moves in the rear direction against the biasing force of the biasing member 830 as shown in Fig. 9(b). As a result, the engaging member 840 is spaced apart from the slit 322 as shown in Fig. 9(b). As a result, the lock of the lid 310 is released and the user can open the lid 310.

As described above, the shaft 820 is biased in the front direction by the biasing member 830. More specifically, in a state that the shaft 820 is moving relative to the rear direction, a curved angle of the curved portion 832 is reduced. In the curved portion 832 in this state, elastic force occurs so as to increase the curved angle. Therefore, when the unlocking device 182 is spaced apart from the inclined portion 810, the locking mechanism 800 returns to the state as shown in Fig. 9(a). Specifically, when the unlocking device 182 is spaced apart from the inclined portion 810, the engaging member 840 protruded by the biasing force of the biasing member 830 is fitted into the slit 322 formed in the lid 310 and engaged with it. As shown in Fig. 9(a), when the lid 310 is closed, it is fitted into the slit 322 by the protrusion of the engaging member 840. As a result, the lid 310 is locked and the user cannot open the lid 310. With this configuration, the cassette can be removed from the medicine loading device and the biasing member 830 serving as the trigger can be returned from the releasing state of the lock to the original state.

§2.3 Advantage of the medicine dispensing cassette 200 In the medicine dispensing cassette 200, whereas the medicine dispensing device 100 can release the lock of the lid 310, the user cannot easily release the lock. Accordingly, when the medicine dispensing cassette 200 is used in the medicine dispensing device 100, it is prevented that the improper medicine is refilled at the time of refilling the medicine into the emptied medicine dispensing cassette 200. This is because the medicine dispensing device 100 scans the bar code attached to the label of the bottle containing the medicine to be refilled and releases the lock of the lid 310 only when the medicine in the bottle is proper as the medicine to be refilled. With this configuration, the specification of the medicine dispensing device 100 of the above embodiment can be a closed loop specification.

The closed loop is a specification for improving the management for the medicines by using an identification device to identify the medicines more accurately and attaching labels or the like to the containers containing the medicines in order to promote the safe use of the medicines and prevent misconception of different medicines. In particular, in the case of recovering the medicines, the recovered medicines can be safely reused by a closed loop recovery for the medicines. Usually, sources of the medicines to be recovered are mainly three sources containing return from the pharmacy, unused medicines in a hospital and return from outpatient visitors. Since distribution routes of the medicines and the states of the medicines recovered from each source generally differ from each other greatly, for example, the unused medicines in the hospital are often recovered in an almost unopened state, while the returned medicines from the outpatient visitors may be opened and damaged. As described above, when there are the plurality of recover routes, it is generally difficult to recover the medicines. In the closed loop specification of the present invention, since recovery processes can be appropriately performed with respect to the medicine dispensing device 100 on the basis of the discrimination information after the discrimination for the medicine has been performed, it is possible to prevent an error of the prescription operator and reduce labor of the prescription operator.

§3 Medicine recovery method A medicine recovery method by the above-described medicine loading device based on the prescription information is shown. Specifically, in the method of the present invention, it has a step of obtaining medicine information indicating a type of a medicine which is a discrimination target, a step of comparing medicine information with cassette information indicating a type of a medicine to be contained in a medicine dispensing cassette, a step of determining whether the medicine information and the cassette information match each other and a step of releasing a locking mechanism provided at the medicine dispensing cassette when the medicine information and the cassette information match each other.

Medicine discriminating means further has a medicine information obtaining part and an information processing part connected to the medicine information obtaining part. The medicine discriminating means reads the cassette information provided at either one of the medicine dispensing cassette and the lid and the medicine information provided on the medicine to discriminate the type of the medicine based on this obtained information on the medicine. Further, the medicine information obtaining part includes at least one of a photographing device for obtaining an image in which information on the medicine which is a discrimination target is indicated, a first reading device for reading a code in which information on the medicine which is the discrimination target is indicated and a second reading device for reading a signal indicating the medicine information on the discrimination target. Hereinafter, as an example of the medicine information obtaining part of the medicine photographing device, description will be given to how to discriminate each medicine in the photographed image in detail.

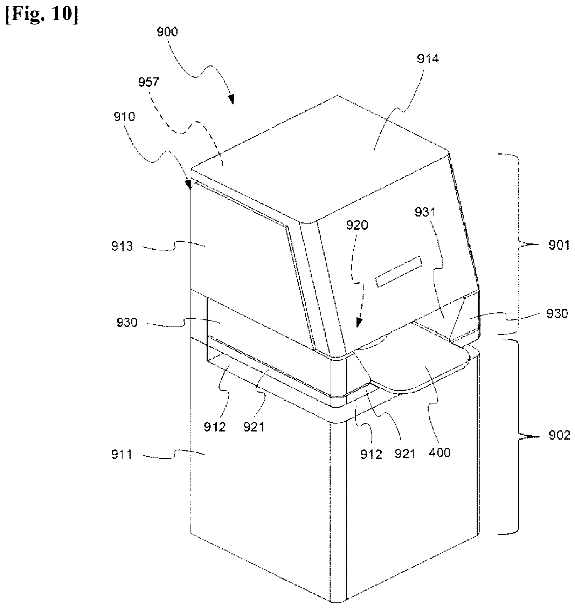

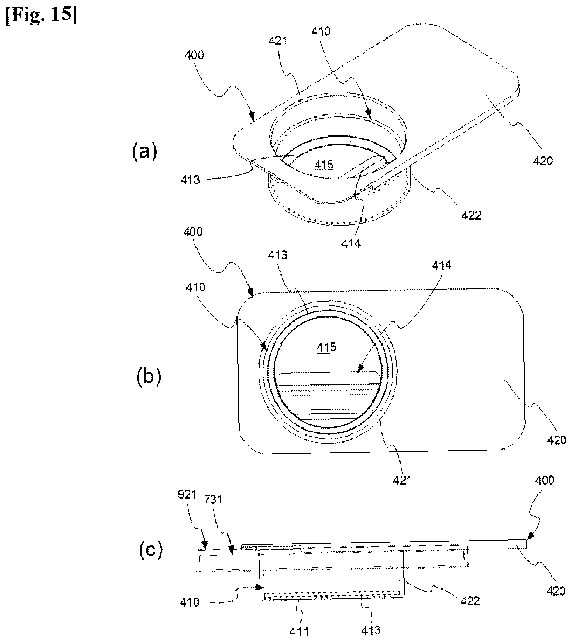

§3.1 Medicine photographing device Fig. 10 is a perspective view showing the appearance of the medicine photographing device 900. Although the inside is not visible by a case 910 in this figure, a placing portion 920 on which the medicine should be placed is provided at a substantially central portion of the inside of the medicine photographing device 900. More specifically, the medicine is placed in a tray 930 as shown in Fig. 15 and this tray 400 is set onto the placing portion 920. Hereinafter, for convenience of explanation, a portion of the medicine photographing device 900 upper than the placing portion 920 is referred to as an upper portion 901 and a portion of the medicine photographing device 900 lower than the placing portion 920 is referred to as a lower portion 902.

§3.1.1 Outer configuration of the medicine photographing device As shown in Fig. 10, a lower case 911, a cutout portion 912, a tray support member 921, guide members 930, an upper case 913 and a cover 914 can be seen from the outside of the medicine photographing device 900.

The lower case 911 covers the lower portion 902 of the medicine photographing device 900. The cutout portion 912 extending in the horizontal direction is formed on an upper surface of the lower case 911. This cutout portion 912 is opened in the left-right direction and the front direction of the medicine photographing device 900. The user can insert long packaging paper into the medicine photographing device 900 through this cutout portion 912. Specifically, the packaging paper is spread in the horizontal direction and inserted into the cutout portion 912 from the front side. Then, the packaging paper is moved into the vicinity of the central portion of the medicine photographing device 900. As a result, a built-in camera of the medicine photographing device 900 can photograph the medicine enclosed in the packaging paper without cutting the packaging paper. Since an internal space of the medicine photographing device 900 communicates with the external space in the left-right direction through the cutout portion 912, even if the packaging paper is longer than a width of the medicine photographing device 900, the user can easily insert the packaging paper into the medicine photographing device 900 in a state that the packaging paper is rewound.

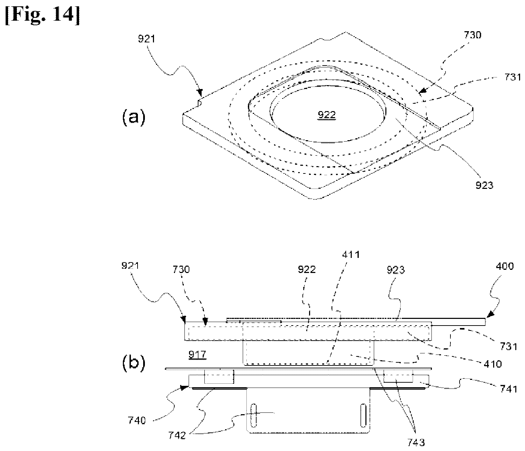

The tray support member 921 for supporting a member for containing the medicines is provided immediately above the cutout portion 912. Specifically, the tray 400 is placed onto the tray support member 921. This tray support member 921 is a substantially square plate-like member in which the circular hole 922 is opened at its central portion as shown in Fig. 14(a). As shown in Fig. 15, a petri dish 410 is fitted in the tray 400. When the tray 400 is set on the tray support member 921, the petri dish 410 is inserted into the hole 922. Further, referring back to Fig. 14(a), a concave portion having a planar shape corresponding to a planar shape of the tray 400 is formed on an upper surface of the placing member 921. An engaging portion 923 into which the tray 400 is fitted is formed by the concave portion. The tray 400 is placed onto this engaging portion 923. Further, when the tray 400 is fitted into the engaging portion 923, the tray 400 is stably held at the tray support member 921.

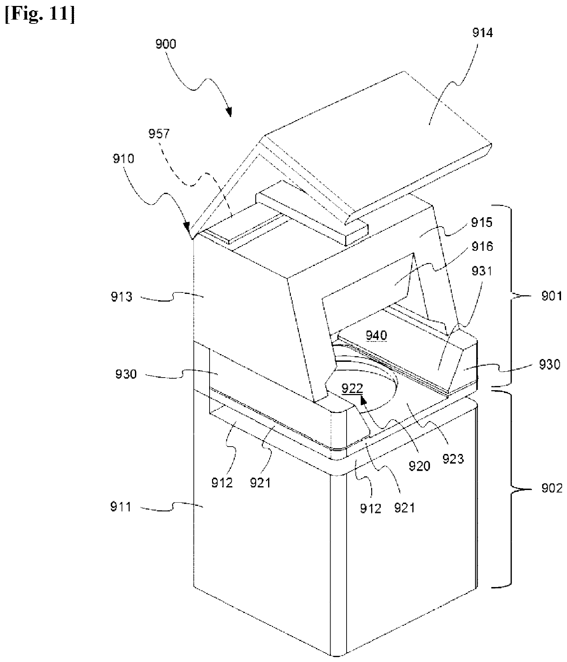

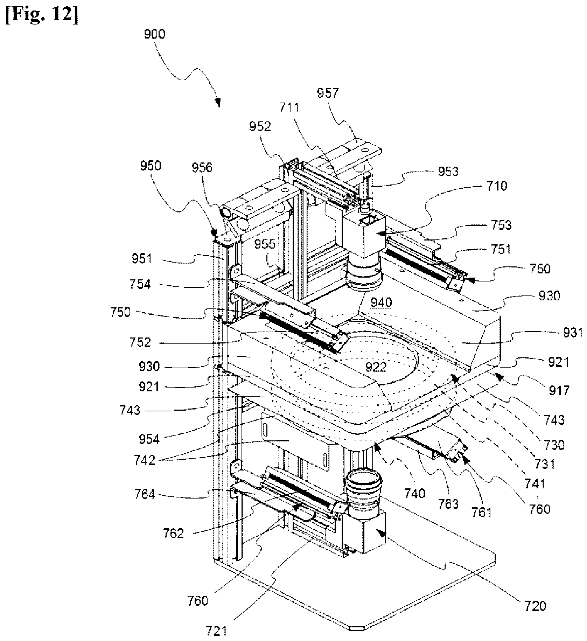

As shown in Fig. 10, the upper surface of the medicine photographing device 900 and the upper portion 901 of the front surface are covered by a cover 914. As shown in Fig. 11, the cover 914 can be opened toward the upper side. Specifically, the cover 914 is hinged by a hinge 957 (see Fig. 12) at a rear upper end of the upper case 913 and the cover 914 can pivot around this hinge 957 in the vertical direction.

Fig. 11 shows a state that the cover 914 of the medicine photographing device 900 is opened. As shown in this figure, when the cover 914 is opened, an upper internal space 940, which is the upper portion 901 of the internal space of the medicine photographing device 900, is opened toward the space in front of the medicine photographing device 900. Then, the user can set the tray 400 onto the engaging portion 923 of the placing portion 920 through this upper internal space 940.

As shown in Fig. 11, a front side outer surface of the upper case 913 has a substantially inverted U shape. This front side outer surface is inclined, thereby an inclined portion 915 is formed. Specifically, the inclined portion 915 is inclined so that a distance from the rear surface of the medicine photographing device 900 becomes small from the lower side to the upper side. With this configuration, the user can easily insert the tray 400 into the upper internal space 940. The user often moves the tray 400 from the obliquely upward side toward the obliquely downward side to insert the tray 400 into the upper internal space 940. At this time, if the inclined portion 915 is inclined toward the rear side as it goes upward, a larger space is secured in front of an upper portion of the inclined portion 915 and thus the tray 400 is less likely to collide with the upper case 913. In this regard, the front surface of the cover 914 is configured to cover this inclined portion 915. Therefore, when the cover 914 is closed, the front surface thereof is located in the vicinity of the inclined portion 915 and becomes parallel to the inclined portion 915. As described above, the upper surface and the front surface of the cover 914 form an obtuse angle in response to the configuration that the upper surface of the upper case 913 and the inclined portion 915 form an obtuse angle.

A lower end of the inclined portion 915 is located on the rear side than outer front surfaces of the guide members 930 and the lower case 911. Namely, a distance from the rear surface of the medicine photographing device 900 to the lower end of the inclined portion 915 is smaller than a distance from the rear surface of the medicine photographing device 900 to the outer front surfaces of the guide members 930 and the outer front surface of the lower case 911. With this configuration, the user can more easily insert the tray 400 into the upper internal space 940.

Furthermore, a slope 916 other than the inclined portion 915 is provided on the front side outer surface of the upper case 913. More specifically, the slope 916 is provided in a U shape of the substantially inverted U-shaped inclined portion 915. This slope 916 is provided in front of a first camera 710 (see Fig. 12) described later to protect the first camera 710 described later to protect the first camera 710. With this configuration, when the user inserts the tray 400 into the upper internal space 940, it is possible to prevent the tray 400 from colliding with the first camera 710, so that it is possible to prevent a position and an orientation of the first camera 710 from being shifted. As shown in Fig. 11, the slope 916 forms a substantially rectangular shape and extends in the left-right direction of the medicine photographing device 900. Further, a distance from the rear surface of the medicine photographing device 900 to the slope 916 gradually decreases from the upper side to the lower side. Namely, a horizontal position of an upper end of the slope 916 is closer to the rear surface of the medicine photographing device 900 than a horizontal position of a lower end of the slope 916. With this configuration, the tray 400 is less likely to collide with the slope 916 at the time of insertion, so that the user can easily put the tray 400 into the rear side of the upper internal space 940.

As shown in Fig. 11, the pair of guide members 930 are provided between the upper case 913 and the placing portion 920. An outer side surface of each guide member 930 extends in the vertical direction and is located on the same plane as the outer side surface of the upper case 913. In contrast, as shown in Figs. 11 to 13, an inner side surface of each guide member 930 is inclined with respect to the vertical direction, thereby slopes 931 are formed. Each slope 931 forms a substantially rectangular shape and extends in the front-rear direction of the medicine photographing device 900. Further, the slopes 931 face the upper internal space 940. Furthermore, one of the slopes 931 is located on the right upper side of the placing portion 920 and the other one is located on the left upper side of the placing portion 920. Each slope 931 is formed so as to approach to a center axis of the medicine photographing device 900 from the upper side to the lower side. Namely, when they are viewed in planar view, a horizontal position of a lower end of each slope 931 is closer to the placing portion 920 than a horizontal position of an upper end of each slope 931. Furthermore, a distance between the lower ends of the two slopes 931 is substantially equal to or slightly larger than a width of the engaging portion 923 of the tray support member 921 (see Fig. 14(a)). Specifically, the distance between the lower ends of the two slopes 931 is preferably about 1 to 1.2 times the width of the engaging portion 923. When the medicine photographing device 900 is viewed in planner view, portions of the lower ends of the slopes 931 are located at positions substantially in contact with a periphery of the hole 922 of the tray support member 921. By employing this configuration, the user can easily place the tray 400 onto the engaging portion 923 of the tray support member 921.

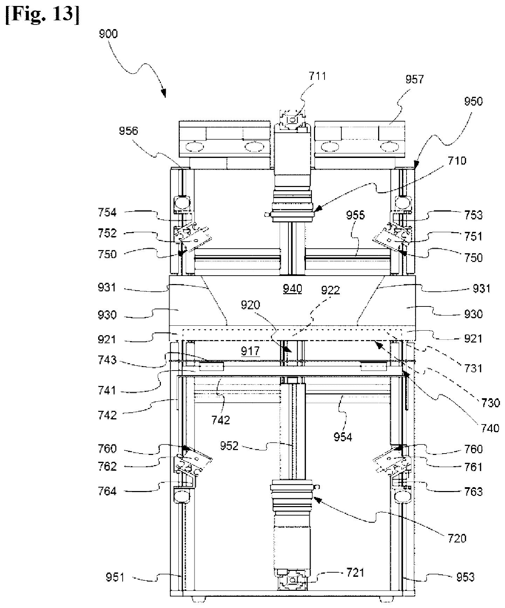

§3.1.2 Internal configuration of the medicine photographing device Figs. 12 and 13 are diagrams showing a state that cases of the medicine photographing device 900 are removed, that is, diagrams showing main members inside the medicine photographing device 900. In this regard, Fig. 12 is a perspective view of the medicine photographing device 900 and Fig. 13 is a front view thereof.

As shown in Fig. 12, a frame 950 is provided on the rear side of the medicine photographing device 900. Many of the main members of the medicine photographing device 900 are configured to be directly or indirectly attached to this frame 950. The frame 950 is constituted of a first column 951, a second column 952, a third column 953, a first beam 954, a second beam 955, a third beam 956 and a hinge 957. When they are viewed in the front view, the first column 951 is provided on the rear left side of the medicine photographing device 900, the second column 952 is provided at a rear central portion of the medicine photographing device 900 and the third column 953 is provided on the rear right side of the medicine photographing device 900. Further, the first column 951, the second column 952 and the third column 953 are coupled by the first beam 954, the second beam 955 and the third beam 956. Furthermore, the hinge 957 for pivoting the cover 914 (see Fig. 3) is attached to the third beam 256 located at a top of the frame 950.

As shown in Figs. 12 and 13, the medicine photographing device 900 includes the first camera 710, a second camera 720, a first light source 730, a second light source 740, a third light source 750, a fourth light source 760, the tray support member 921 and the guide members 930. Among them, the tray support member 921 and the guide members 930 are directly attached to the frame 950. The first camera 710, the second camera 720, the third light source 750 and the fourth light source 760 are attached to the frame 950 through support members. The first light source 730 is contained in the tray support member 921 and supported by the tray support member 921. Although this matter is not shown in the figures, the second light source 940 is attached to the lower case 911 (see Fig. 10) through the support member. As described above, the main members inside the medicine photographing device 900 are attached to the frame 950 instead of the case 910. With this configuration, it becomes easier to remove the case 910 and thus maintenance work of the inner main members becomes easy.

The tray support member 921 is provided in the vicinity of the central portion of the medicine photographing device 900. Further, the guide members 930 are provided immediately above the tray support member 921. The third light source 750 is provided on the upper side of the guide members 930. Furthermore, the first camera 710 is provided at a position higher than the third light source 750, that is, in the vicinity of the upper surface of the medicine photographing device 900. The second light source 740 is provided on the lower side of the tray support member 921 with being apart from the tray support member 921 by a predetermined distance. The fourth light source 760 is provided at a position lower than the second light source 740 and in the vicinity of the side surface of the medicine photographing device 900. The second camera 720 is provided at a position lower than the fourth light source 760, that is, in the vicinity of the bottom surface of the medicine photographing device 900. As described above, in the medicine photographing device 900, an upper light source is constituted of the first light source 730 and the third light source 750. Further, in the medicine photographing device 900, a lower light source is constituted of the second light source 740 and the fourth light source 760.

As described above, the tray support member 921 is provided in the vicinity of the central portion of the medicine photographing device 900 and on the upper side of a space 917 for the cutout portion 912. As shown in Fig. 14(a), the engaging portion 923 is formed on the upper surface of this tray support member 921 and the tray 400 (see Fig. 15) is placed onto this engaging portion 923 in use. Further, the first light source 730 is attached to a bottom portion of the tray support member 921. The first light source 730 includes a first ring light 731. This first ring light 731 is constituted by a plurality of light emitting diodes (LED) annularly arranged. The plurality of LEDs are arranged so as to surround an outer periphery of the hole 922 of the tray support member 921 and face the hole 922.

As shown in Fig. 14(b), when the tray 400 is placed onto the engaging portion 923, the petri dish 410 passes through the hole 922 and protrudes toward the lower side of the tray support member 921. Therefore, a bottom portion 411 of the petri dish 410 is located on the lower side than the lower surface of the tray support member 921. In other words, when the tray 400 is placed onto the engaging portion 923, the petri dish 410 is inserted into the ring of the first ring light 731 and the bottom portion 411 of the petri dish 410 is located on the lower side than the first ring light 731. Namely, when the tray 400 is placed onto the engaging portion 923, the first ring light 731 is located at a position higher than the bottom portion 411 of the petri dish 410 and lower than an upper surface or an upper end of the tray 400 (the highest position of the tray 400). At least a bottom surface and a side surface, which faces the hole 922, of the tray support member 921 are made of a transparent material. As a result, when the first ring light 731 emits light, that emitted light propagates in a direction toward the center of the hole 922 and the lower side. As a result, the medicine supplied onto the bottom portion 411 of the petri dish 410 is directly illuminated by the light emitted from the first ring light 731 from all directions of 360° and the obliquely upward side. Namely, the first light source 730 serves as a direct light source for directly illuminating the medicine.

As shown in Fig. 14(b), the second light source 740 is provided on the lower side of the tray support member 921 through the cutout space 917. The second light source 740 is constituted of a second ring light 741 which is the same type as the first ring light 731. This second ring light 741 is supported by a lower support plate 742 and an upper support plate 743. The lower support plate 742 and the upper support plate 743 are attached to an inner surface of the lower case 911 (see Fig. 10). The upper support plate 743 is made of a transparent material. Further, the upper support plate 743 has a substantially rectangular planar shape and does not have a hole in its central portion (on the lower side of the hole 922) unlike the tray support member 921. With this configuration, even if the user mistakenly drops the medicine into the placing portion 920, it is possible to prevent the dropped medicine from colliding with the second camera 720. The lower support plate 742 has a planar shape corresponding to a planar shape of the second ring light 741 and has a hole in its central portion, which has a shape corresponding to the ring of the second ring light 741. The second ring light 741 is located on the lower side than the bottom portion 411 of the petri dish 410. Further, a planar position of the second ring light 741 is arranged so as to match a planar position of the first ring light 731. When the second ring light 741 emits light, this emitted light propagates in the direction toward the center of the hole 922 and the upper side. As a result, the medicine supplied onto the bottom portion 411 of the petri dish 410, the medicine is directly illuminated by the light emitted by the second ring light 741 from all directions of 360° and the obliquely downward side. The first light source 730 and the second light source 740 described above are mainly used for photographing the engraved marks attached to the surfaces of the medicine, mainly a tablet. More specifically, the first light source 730 is used for photographing the engraved mark positioned on the upper surface of the medicine and the second light source 740 is used for photographing the engraved mark positioned on the lower surface of the medicine. The present inventor has found that it is possible to clearly photograph the engraved marks attached to the surfaces of the tablet by configuring the first light source 730 and the second light source 740 as described above.

As shown in Figs. 12 and 13, the third light source 750 is provided on the upper side of the guide members 930. This third light source 750 is constituted of a plurality of bar light, specifically, a first bar light 751 and a second bar light 752. The first bar light 751 is attached to the third column 953 through a support member 1053. Further, the second bar light 752 is attached to the first column 951 through a support member 1054. In other words, the first bar light 751 and the second bar light 752 are provided on the lateral side of the medicine photographing device 900, specifically, on the lateral side than the placing portion 920. As shown in Fig. 13, the first bar light 751 and the second bar light 752 are provided in a direction parallel to the front-rear direction of the medicine photographing device 900 and light emitting surfaces of the first bar light 751 and the second bar light 752 are directed obliquely downward, more specifically, toward the placing portion 920. Further, a distance from the first bar light 751 and the second bar light 752 to the place of the placing portion 920 where the medicine should be placed is larger than a distance from the first ring light 731 to the place of the placing portion 920 where the medicine should be placed. Furthermore, the first bar light 751 and the second bar light 752 are provided at the lower ends of the slopes 931 of the guide members 930 and at a position closer to the side surface of the medicine photographing device 900 than the lower ends of the slopes 931 of the guide members 930 and the outer periphery of the hole 922 of the tray support member 921. With this arrangement of the third light source 750, a large space is secured by the upper internal space 940 and thus the user can easily place the tray 400 onto the placing portion 920.

Each of the first bar light 751 and the second bar light 752 includes a polarization filter and the light diffuses through this polarization filter and reaches the medicine. Specifically, it is configured so that direct light from the first bar light 751 and the second bar light 752 is cut by the polarization filters and the light diffused by the polarization filters reaches the medicine through the hole 922 of the tray support member 921, that is, the inside of the ring of the first ring light 731. Namely, the third light source 750 serves as a diffused light source or an indirect light source. Thus, the upper portion of the medicine is suitably illuminated. The present inventor has found that it is possible to clearly photograph the print attached to the surface of the medicine by configuring the third light source 750 as described above. Furthermore, regardless of whether the medicine is a tablet or a capsule, it is possible to clearly photograph the print.

As shown in Figs. 12 and 13, the fourth light source 760 which is the same type as the third light source 750 is provided on the lower side of the second light source 740. This fourth light source 760 is constituted of a plurality of bar light, specifically, a third bar light 761 and a fourth bar light 762. The third bar light 761 is attached to the third column 953 through a support member 1063. Further, the fourth bar light 762 is attached to the first column 951 through a support member 1064. In other words, similarly to the first bar light 751 and the second bar light 752, the third bar light 761 and the fourth bar light 762 are provided on the lateral side of the medicine photographing device 900. Furthermore, similarly to the first bar light 751 and the second bar light 752, the third bar light 761 and the fourth bar light 762 are provided in the direction parallel to the front-rear direction of the medicine photographing device 900. Light emitting surfaces of the third bar light 761 and the fourth bar light 762 are directed obliquely upward, more specifically, toward the placing portion 920. Similar to the relationship between the third light source 750 and the first light source 730, a distance from the third bar light 761 and the fourth bar light 762 to the placing portion 920 is larger than a distance from the second ring light 741 to the placing portion 920. Similar to the first bar light 751 and the second bar light 752, each of the third bar light 761 and the fourth bar light 762 also includes a polarization filter and configured so that light diffuses through the polarization filters and reaches the medicine. More specifically, it is configured so that the diffused light from the third bar light 761 and the fourth bar light 762 passes through the inside of the ring of the second ring light 741 and reaches the medicine. With this configuration, the lower portion of the medicine is illuminated to make its print clear.

In this regard, although each of the third light source 750 and the fourth light source 760 is constituted of the two bar lights in the present embodiment, four bar lights may be provided in other embodiments. In this case, it is preferable to arrange the bar lights so that the four bar lights form a square shape and surround the placing portion 920.

The first camera 710 is provided on the upper side of the first bar light 751 and the second bar light 752. This first camera 710 is attached to the second column 952 through an attachment member 1011. Then, the first camera 710 is fixed by this attachment member 1011 so as to be located vertically above the placing portion 920 and face the placing portion 920. When the medicine photographing device 900 is viewed in the planar view from the upper side, a photographing area of the first camera 710 contains the hole 922 of the tray support member 921 and the inside of the ring of the first ring light 731. Thus, the first camera 710 can suitably take an image of the upper surface of the medicine placed on the placing portion 920, that is, an image of the medicine viewed from directly above.

The second camera 720 is provided on the lower side of the third bar light 761 and the fourth bar light 762. This second camera 720 is attached to the second column 952 through an attachment member 1021. Then, the second camera 720 is fixed by this attachment member 1021 so as to be located vertically below the placing portion 920 and face the placing portion 920. When the medicine photographing device 900 is viewed in the planar view from the lower side, a photographing area of the second camera 720 contains the inside of the ring of the second ring light 741. Thus, the second camera 720 can suitably take an image of the lower surface of the medicine placed on the placing portion 920, that is, an image of the medicine viewed from directly below. Both of the first camera 710 and the second camera 720 described above can take a color image.

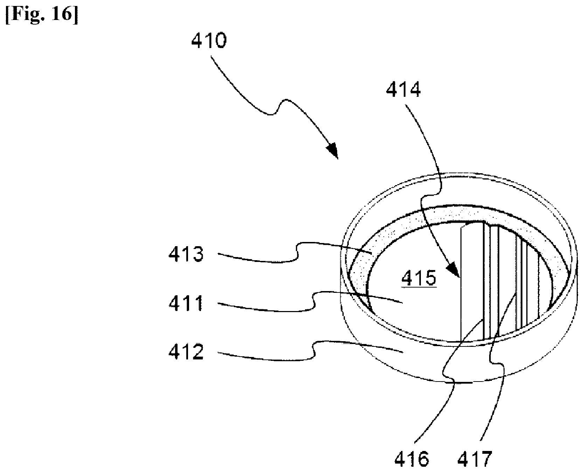

§3.1.3 Configuration of placing item The tray 400 as shown in Fig. 15 is used as a placing item for placing the medicine thereon in the medicine photographing device 900. Tray 400 has a configuration in which a hole 421 is formed in a main body 420 having a square flat plate-like shape and a transparent cylinder 422 is fitted in the hole 421. The petri dish 410 as shown in Fig. 16 is provided in this cylinder 422.

Fig. 16 shows the petri dish 410. The bottom surface 411 and the side surface 412 of this petri dish 410 are transparent. An opaque and flat ring 413 is provided on an outer edge of the bottom surface 411. When the medicine rolls into the vicinity of the inner wall of the petri dish 410, the illumination light is diffusely reflected by this medicine and this reflection of the light adversely affects the photographing of the medicine in the vicinity. The ring 413 can suitably prevent the medicine from rolling into the vicinity of the inner wall. Furthermore, the ring 413 can reduce the unwanted reflection of the illumination light occurring in the vicinity of the boundary between the bottom surface 411 and the inner wall of the petri dish 410. From such a point of view, it is preferable that this ring 413 is black. A width of this ring 413 is preferably about 1/20 to 1/2 times a radius of the bottom surface 311 of the petri dish 310.

Further, a capsule placing member 414 which is preferably made of a black opaque material is provided on the bottom surface 411 of the petri dish 410. An approximately half of the bottom surface 411 of the petri dish 410 is occupied by the capsule placing member 414 and the transparent material constituting the bottom surface 411 is exposed in the other half thereof. A portion of the bottom surface 411 where this transparent material is exposed is referred to as a transparent portion 415. It is recommended that the tablet should be placed on this transparent portion 415. On the other hand, it is recommended that capsule types such as capsules and soft capsules should be placed on the capsule placing member 414 in principle. The capsule placing member 414 has a planar shape close to a trapezoidal shape. Specifically, the capsule placing member 414 has a long side and a short side in a lengthwise direction thereof and has two hypotenuse sides having substantially the same length in the transverse direction. A distance between hypotenuse sides thereof decreases from the long side toward the short side. By setting the planar shape of the capsule placing member 414 to such a shape, it becomes possible to place more the capsules per part area. When the capsule placing member 414 is viewed from the lateral side, the capsule placing member 414 has a portion having a wavy shape on its upper surface. In other words, the capsule placing member 414 has groove-shaped concave portions 416 and 417 on its upper surface. A width and a depth of the concave portion 416 are set to be larger than a width and a depth of the concave portion 417. Further, a length of the concave portion 416 is larger than a length of the concave portion 417. Thus, a relatively large capsule can be easily placed in the concave portion 416 and a relatively small capsule can be easily placed in the concave portion 417. The capsule is placed in the concave portions 416 or 417 so that the printed characters of the capsule are directed toward the upper side. Since the concave portions 416 or 417 prevents the capsule from rolling, the first camera 710 can accurately photograph the print characters of the capsule.

As described above, the tray 400 is placed onto the tray support member 921 (see Fig. 14). As shown in Fig. 15(c), when the tray 400 is placed onto the tray support member 921, the first ring light 731 is located in the vicinity of the upper portion of the cylinder 422. In other words, the first ring light 731 is located in the vicinity of the upper obliquely side of the bottom surface 411 of the petri dish 410, that is, located in the vicinity of the bottom surface 411 and on the upper side and the lateral side of the bottom surface 411. The present inventor has found that it is possible to illuminate the medicine so that the engraved mark of the tablet can be more clearly photographed by placing the first ring light 731 at the above-described position. In this regard, as shown in Fig. 14, a height difference from the second ring light 741 to the bottom surface 411 of the petri dish 410 is set to be substantially equal to a height difference from the first ring light 731 to the bottom surface 411 of the petri dish 410. From such a point of view, it is preferable that the height difference from the second ring light 741 to the bottom surface 411 of the petri dish 410 is about 0.5 to 2 times the height difference from the first ring light 731 to the bottom surface 411 of the petri dish 410.

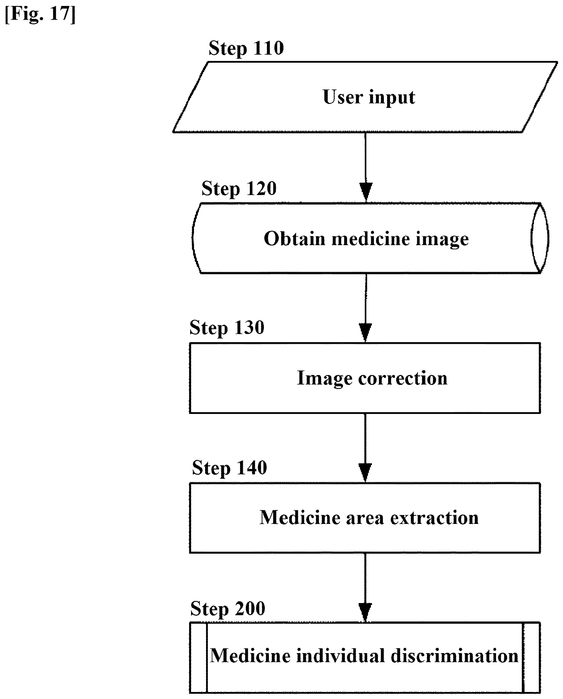

§3.2 Medicine discrimination Fig. 17 shows upstream processes for performing medicine discrimination. First, when the user supplies the medicines as an input to start the medicine discrimination (step 110), the medicine photographing device takes a plurality of images of the medicines (step 120). Next, the computer (the information processing part) corrects the photographed images (step 130). Next, the computer extracts an area in which each medicine exists from the corrected images (step 140). Then, discrimination is individually performed on the extracted area of each medicine (step 200).

<Step 110> Medicine supplying The user first places the medicines to be discriminated in the petri dish of the tray of the medicine discriminating means. For example, it is recommended that the tablet should be placed directly on the transparent portion of the bottom surface of the petri dish and the capsule should be placed on the capsule placing member. Next, the user places this tray onto the placing portion of the medicine photographing device.

<Step 120> Medicine image obtaining When the computer receives an input to start to photograph the medicines from the user, the computer drives the medicine photographing device to take the image of the petri dish. Specifically, the computer obtains four images of the following "image 1" to "image 4". At this time, the computer sets photographing conditions of the camera based on setting values (e.g., a focus of the camera, an exposure time and each gain of RGB) obtained at steps of "focus adjustment of the camera" and "brightness and color calibration" described later. This improves the reproducibility of the image quality. [Image 1] The image 1 is an image obtained by photographing the petri dish from the upper side under the illumination by the first light source. This image can be obtained by turning on the first ring light and performing photographing with the first camera. [Image 2] The image 2 is an image obtained by photographing the petri dish from the lower side under the illumination by the second light source. This image can be obtained by turning on the second ring light and performing photographing with the second camera. [Image 3] The image 3 is an image obtained by photographing the petri dish from the upper side under the illumination by the third light source. This image can be obtained by turning on the first bar light and the second bar light and performing photographing with the first camera. [Image 4] The image 4 is an image obtained by photographing the petri dish from the lower side under the illumination by the fourth light source. This image can be obtained by turning on the third bar light and the fourth bar light and performing photographing with the second camera.

<Step 130> Image correction As described later, in the medicine discriminating means, calibration is performed in advance and the computer holds calibration information obtained by this calibration as data. The computer corrects the photographed images based on this calibration information. Specifically, correction of coordinates of the photographed images is performed based on the calibration information. Thus, coordinate axes of the images photographed by the first camera coincide with coordinate axes of the images photographed by the second camera.

<Step 140> Medicine area extraction After the image correction, an area occupied by each medicine is extracted in the images. For this purpose, the "image 1" and the "image 2" are suitably used, each of which has a tendency that the brightness greatly changes between the background and the medicine. This area extraction is performed by binarizing the brightness of each pixel with a threshold value (below the threshold value=background, over the threshold value=medicine) and separating areas over the threshold value.

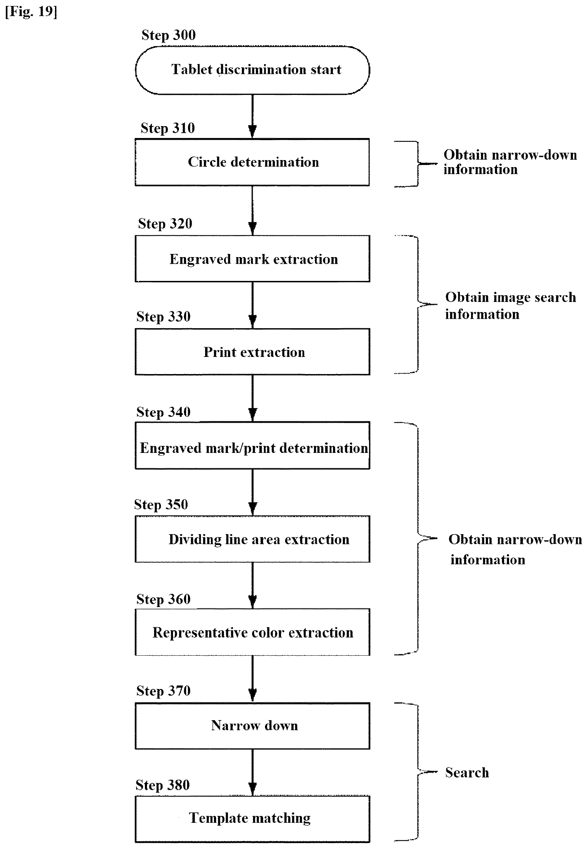

Fig. 19 illustrates a flow of a medicine individual discrimination process 200. The following processes are performed individually for each medicine. First, the computer associates the occupied area of one medicine in the photographed image captured from the upper side with the occupied area of this medicine in the photographed image captured from the lower side (step 210). Next, the computer determines whether the medicine is a tablet or a capsule (step 220). When it is determined that the medicine is the tablet, the computer performs a tablet discrimination process (step 300) and when it is determined that the medicine is the capsule, the computer performs a capsule discrimination process (step 400) .

<Step 210> Association of the medicines in the upper and lower images The medicine directly placed on the transparent portion of the petri dish appears in both of the images captured from the upper side (the "image 1" and the "image 3") and the images captured from the lower side (the "image 2" and the "image 4"). Due to this step, the area occupied by the one medicine in the image captured from the upper side and the area occupied by this medicine in the image captured from the bottom side are associated with each other. Specifically, when one image of the "image 1" and the "image 2" corrected at step 130 is reversed horizontally or vertically, the area occupied by the medicine in one of the images substantially matches the area occupied by the same medicine in the other one of the images. This matched area is an area occupied by the one medicine in each of the two images. Namely, one area in the non-inverted image and an area obtained by returning the area in the inverted image overlapping with it due to the inversion release are areas respectively corresponding to the one medicine. At this time, an overlapping area in which the two images are overlapped with each other when the two images are inverted may be used as a corresponding area.

<Step 220> Tablet/capsule determination After the association process for the medicine area has been performed, the computer determines whether the medicine is a tablet or a capsule. Since the tablet is placed onto the transparent portion of the petri dish, the upper surface of the tablet appears in the image captured from the upper side and the lower surface of the tablet appears in the image captured from the lower side. Namely, when the two images (the "image 1" and the "image 2") are compared, if the medicine is a tablet, an area corresponding to the medicine exists in each of the image captured from the upper side and the image captured from the lower side. Therefore, in the case where the association for one area with respect to the two images can be performed at step 210, namely, in the case where the occupied area of the medicine recognized in the image captured from the upper side is associated with the occupied area of the medicine recognized in the image captured from the lower side, the medicine is determined as the tablet.

On the other hand, the capsule is placed on the opaque capsule placing member. Thus, although the upper surface of the capsule appears in the image captured from the upper side, the capsule does not appear in the image captured from the lower side. Namely, when the two images (the "image 1" and the "image 2") are compared, if the medicine is the capsule, whereas an area occupied by the medicine exists in the image captured from the upper side, an area occupied by the medicine does not exist in the image captured from the lower side. Therefore, in the case where the association for one area with respect to the two images at step 210 cannot be performed, namely, in the case where the area corresponding to the occupied area of the medicine recognized in the image captured from upper side is not recognized in the image captured from the lower side, the medicine is determined as the capsule.

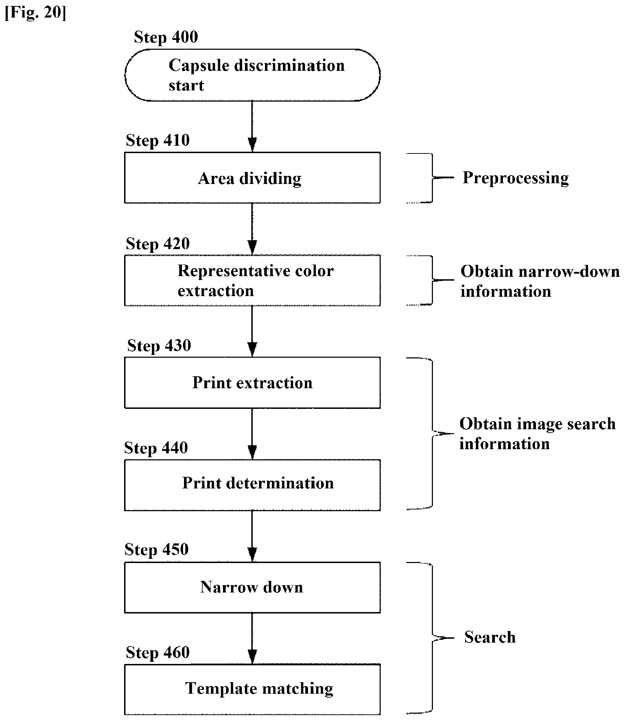

<Step 300> Tablet discrimination If one medicine selected by the computer is determined as the tablet, the process proceeds to a tablet discrimination process 300. Fig. 20 shows each process of the tablet discrimination process 300. First, the computer determines whether or not the medicine is circular (step 310). Next, the computer performs an extraction process for the engraved mark attached to the surface of the medicine (step 320). Next, the computer performs an extraction process for the print attached to the surface of the medicine (step 330). Next, the computer determines whether or not the information attached to the surface of the medicine is the engraved mark or the print (step 340). Next, the computer extracts a dividing line attached to the surface of the medicine (step 350). Next, the computer extracts a representative color of the medicine (step 360). Thereafter, the computer narrows down candidate medicines for the medicine based on narrow-down information on the medicine (step 370). Finally, the computer performs template matching based on the engraved mark and/or the print information extracted from the images of the medicine and performs a final search (step 380).

<Step 310> Circle determination The computer calculates a circularity of the area occupied by the medicine. If the circularity is equal to or more than a predetermined value, the computer determines that the medicine is circular. When the circularity is less than the predetermined value, the computer determines that the medicine is non-circular. If the medicine is determined to be circular, the computer calculates a center position and a radius of the circle. If the medicine is determined to be non-circular, the computer rotates the area occupied by the medicine so that a long axis of the area occupied by the medicine is parallel to the X axis and a short axis of the area is parallel to the Y axis. In addition, the computer calculates lengths of the long axis and the short axis of the medicine and a ratio of the lengths of the long axis and the short axis of the medicine.

<Step 320> Engraved mark extraction The computer performs an engraved mark extraction process on the "image 1" and the "image 2", that is, the images captured under the illumination of the first light source and the second light source. The present inventor has found that it is possible to capture the image of the medicine in which the engraved mark is emphasized when the medicine is photographed under the illumination of the first light source and the second light source.

<Step 330> Print extraction The computer performs a print extraction process on the "image 3" and the "image 4", that is, the images captured under the illumination of the third light source and the fourth light source. The present inventor has found that it is possible to capture the image of the medicine in which the print is emphasized when the medicine is photographed under the illumination of the third light source and the fourth light source.

<Step 340> Engraved mark/print determination As described above, an image in which the engraved mark attached to the surface of the medicine is suitably extracted can be obtained by performing the engraved mark extraction process (step 320) on the images captured under the illumination of the first ring light and the second ring light. Further, an image in which the print attached to the surface of the medicine is suitably extracted by performing the print extraction process (step 330) on the images captured under the illumination of the first bar light, the second bar light, the third bar light and the fourth bar light (see the above equation 1). On the basis of these engraved mark extracted image and print extracted image, the computer determines whether the information attached to the surface of the medicine is the engraved mark or the print, that is, whether or not the engraved mark and the print are attached to the surface of the medicine.

In order to determine whether or not the engraved mark is attached to the medicine, the computer first performs the binarization process on the engraved mark extracted image obtained at step 320 with using a predetermined threshold value as a criterion. Next, the computer takes an average value of the values of the engraved mark extracted image with using this binarized image as a mask. When the average value is equal to or larger than the threshold value, the computer determines that the engraved mark exists and when the average value is smaller than the threshold value, the computer determines that the engraved mark does not exist. Similarly, in order to determine whether or not the print is attached to the medicine, the computer first performs the binarization process on the print extracted image obtained at step 330 with using on a predetermined threshold value as a criterion. Next, the computer takes an average value of the values of the print extracted image with using the binarized image as a mask. When the average value is equal to or larger than the threshold value, the computer determines that the print exists and when the average value is smaller than the threshold value, the computer determines that print does not exist.

<Step 350> Dividing line area extraction The computer performs a dividing line area extraction process on the engraved mark extracted image obtained at step 320. Specifically, the computer determines whether or not the dividing line exists on the medicine. When it is determined that the dividing line exists, the medicine is divided into an area where the dividing line exists and an area other than it.

<Step 360> Representative color extraction Next, the computer separately extracts the representative color of the medicine for each of the area where the engraved mark or the print is attached and the area other than it. The representative color extraction can be suitably performed by performing clustering on colors constituting the pixels. In addition, in another embodiment, instead of extracting the representative color, a determination for a color density of "white", "light color" and "dark color" is performed on the image of the medicine, or the area where the engraved mark or the print is attached and the area other than it. This can be performed by determining to be "dark color" if the color in the area is less than a first threshold value, the "light color" if it is equal to or greater than the first threshold value and less than a second threshold value and the "white" if it is equal to or greater than the second threshold value.

<Step 370> Narrowing down The computer accesses the database. Then, a search for the candidate medicines is performed based on the data obtained at the above steps. First, as a first step, narrowing down with respect to search target medicines is performed. Specifically, the computer narrows down the search target medicines based on at least one of the following: the type of the medicine (tablet or other type medicine), the shape of the medicine (circular or non-circular, or the ratio of the lengths of the long axis and the short axis in the case of non-circular), the size of the medicine (the diameter of the medicine in the case of circular, the length of the long axis and the length of the short axis of the medicine in the case of non-circular), the presence or absence of the engraved mark, the presence or absence of the print, the presence or absence of the dividing line, the representative color of the medicine (the representative color of the area where the engraved mark is attached, the representative color of the area where the print is attached and the representative color of the area other than it). In this regard, if the computer can access a prescription history of the patient (for example, the data of the medicine handbook), the computer can limit the search target medicines to medicines contained in the prescription history. For example, if the computer can access the data of the medicine handbook stored in a smart phone of the patient, the computer can access the data of the medicine handbook to obtain information on the medicine prescribed to the patient. When there is a medicine handbook printed on paper, the medicine handbook is scanned and converted into text data by OCR (optical character recognition), and the search target medicines can be narrowed down based on the text data. As a result, the speed of the medicine search can be increased.

<Step 380> Template matching Thereafter, the template matching (also referred to as pattern matching) is performed between the engraved marked extracted image and/or the print extracted image obtained at the aforementioned steps and an engraved mark template image and/or a print template image for each medicine stored in the database to pick up medicines having a high possibility of matching with the discrimination target medicine. In this regard, in the case where the tablet is circular, the area of the image occupied by the medicine is moved and corrected so that the center of the circle coincides with the center of the image prior to the template matching. In another embodiment, the extracted image and the template image may be corrected so that the center of gravity of the engraved mark and/or the print coincides with the center of the image. Further, when the tablet is circular, the extracted image and/or the template image is rotated so that the result of the template matching is maximized. Thereafter, the template matching is performed on both images to calculate a score. This score indicates a degree of coincidence of the both images as a numerical value. When the score of the template matching is equal to or larger than a predetermined value or when the score is within a predetermined rank from the top rank, the computer picks up the medicine to which the template image belongs as a candidate medicine. In this regard, when the tablet has the dividing line, both of the template matching in a state that the dividing line exists and the template matching in a state that the dividing line is removed may be performed. Further, when the print or the engraved mark is constituted of words, the computer may perform the template matching on each word as one unit or may divide each word into characters, numbers and symbols to perform the template matching on each character, number and symbol. Further, the computer may extract text information from the engraved mark or the printed image with OCR and perform the template matching based on this text information.

<Step 400> Capsule discrimination Referring back to step 220 in Fig. 19, if the one medicine selected by the computer is determined to be the capsule, the process proceeds to a capsule discrimination process 400. Fig. 20 shows each process of the capsule discrimination process 400. First, the computer divides the capsule into two areas (step 410). Next, the computer extracts a representative color from each of the two areas (step 420). Next, the computer performs an extraction process for the print attached to the surface of the medicine from each of the two areas (step 430). Next, the computer determines whether or not the print is attached to the surface of the medicine (step 440). Thereafter, the computer narrows down the candidate medicines based on the narrow-down information on the medicine (step 450). Finally, the computer performs the template matching based on the print information extracted from the image of the medicine to perform a final search (step 460).

<Step 410> Area dividing First, the computer divides the medicine into the two areas. In particular, the color information of the image of the medicine, specifically, the colors of the pixels are clustered. Next, the clustered color information is grouped and an area to which this color group belongs is categorized as one area. Thus, the capsule whose color is constituted of different colors on a right half side and a left half side thereof is suitably divided into the two areas.

<Step 420> Representative color extraction Next, the computer extracts the representative color of each area of the medicine. This can be suitably performed by clustering the color information of the pixels constituting each area.

<Step 430> Print extraction The computer performs the same process as that of step 330 on each area divided at step 410 to extract the print from each area. The present inventor has found that it is possible to more suitably extract the print from the capsule by dividing the image of the capsule into the two areas and separately performing the extraction process for the print image on these areas.

<Step 440> Print determination The computer performs the same process as that of step 340 on each divided area of the medicine to determine whether or not the print is attached to each area.

<Step 450> Narrowing down The computer narrows down the candidate medicines with the same manner as that of step 360. Specifically, the computer narrows down the search target medicines based on at least one of the type of the medicine (capsule or other type medicine), the shape of the medicine (the ratio of the lengths of the long axis and the short axis of the medicine), the size of the medicine (the lengths of the long axis and the short axis of the medicine), the representative color and the presence or absence of the print in the areas to which each representative color belongs.

<Step 460> Template matching The computer cuts out a printed portion from the print extracted image (input image) obtained at step 430. Next, the template matching is performed on the print extracted image for each area of the print extracted image divided at step 410 with the same manner as that of step 370 to pick up the candidate medicines.

§4 Additional process of medicine discrimination software §4.1 Additional process at "step 370" At step 370 described above, it is assumed that the template image corresponding to the engraved mark extracted image and/or the print extracted image is stored in the database accessed by the computer. In some cases, however, only a photograph of the medicine may be stored. In that case, an area of the medicine may be obtained from this image data and the print extraction and the engraved mark extraction may be performed in the same manner as the above-described case to use the obtained print extracted image and the obtained engraved mark extracted image as the template image. Further, this method can be used to create a template image to be stored in the database. Note that the area of the medicine can be obtained from the image data as follows. First, a background color is obtained and a portion other than the background color and characters is determined to be an area occupied by the medicine. If one or three or more areas are found, it is determined as an error. Next, a position of the center of gravity of each area is obtained and the right side and left side of the medicine are determined from the position of the center of gravity.

§4.2 Calibration for camera In order to suitably practice the above embodiment, the first camera and the second camera need to be in focus. Further, it is necessary to coincide the photographing area of the first camera with the photographing area of the second camera. Furthermore, the brightness and the color of the photographed image should also be within a predetermined value range. For this reason, the calibration as described below is performed before shipping the medicine discriminating system from a factory or at the time of setting up the medicine discriminating system at a delivery destination. Note that once this calibration is performed, it is usually not necessary to perform the calibration again.

§4.2.1 Camera positioning In this calibration, camera positioning is first performed. First, the tray on which the petri dish is set is set onto the placing portion 220 of the medicine photographing device 200. Next, the petri dish is photographed by the first camera and this photographed image is displayed on the display device of the computer. At this time, a virtual image having a cross mark at a predetermined position is superimposed on the image of the petri dish and displayed. Then, the position and the angle of the first camera are finely adjusted so that this cross mark is located inside the petri dish. The same operation is performed for the second camera.

§4.2.2 Camera focus adjustment Next, the focus of the camera is adjusted. First, a template with predetermined characters, symbols or diagrams is set onto the placing portion of the medicine photographing device and this template is photographed with a low magnification. Then, the focus of the camera is roughly adjusted so that the indications of the template becomes the clearest in the photographed image. After this operation has been completed, the magnification is increased and the same operation is performed to finely adjust the focus.

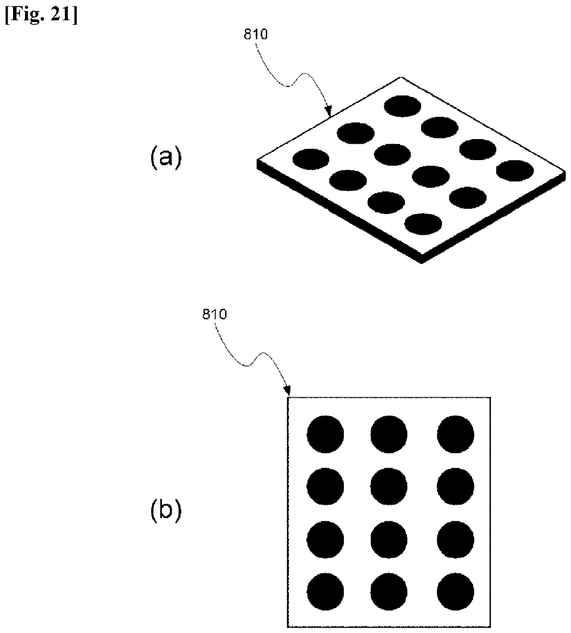

§4.2.3 Calibration of photographing area The coordinates of the photographing areas of the upper and lower cameras can be matched by this calibration. Further, the resolutions of the cameras can also be obtained. First, a calibration sheet 910, which is a calibration tool as shown in Fig. 21, is prepared. This calibration sheet 910 has a configuration in which black circles each having a predetermined size are arranged in a m x n matrix (3 x 4 matrix in the example shown in Fig. 21) at predetermined intervals on both surfaces of a plate or a sheet having a predetermined thickness. Further, positions and sizes of the black circles are the same on both of an upper surface and a lower surface of the calibration sheet 910. At the time of the calibration, this calibration sheet 910 is set onto the placing portion of the medicine photographing device and the calibration sheet 910 is photographed by the first camera and the second camera. Next, a transformation matrix for transforming so that each of the black circles in the image is located at a designated coordinate and has a designated size is calculated. This operation is performed on both of an image captured by photographing the calibration sheet 910 from the upper side and an image captured by photographing the calibration sheet 910 from the lower side. At step 130 described above, correction for the image obtained by photographing the petri dish is performed based on this transformation matrix. With this operation, the coordinate positions of the photographing areas of the image captured by photographing the petri dish from the upper side and the image captured by photographing the petri dish from the lower side coincide with each other. Further, from the transformation matrix, resolution (DPI) of the image, i.e., the number of pixels constituting the part length is also obtained.

§4.2.4 Calibration of brightness and color The color reproducibility of the photographed image can be enhanced by this calibration. In the same manner as the case described above, the following (a) to (b) are repeatedly performed in a state that the calibration sheet 910 is placed on the placing portion. (a) Exposure adjustment: Photographing is performed by the first camera with a predetermined exposure time in a state that the first light source is turned on. Further, photographing is performed by the second camera with a predetermined exposure time in a state that the second light source is turned on. Then, the brightness of each predetermined point of the photographed image is calculated. If this brightness is higher than a target value, the exposure time of the camera is decreased. If this brightness is lower than the target value, the exposure time of the camera is increased. (b) Color (white balance) adjustment: the RGB value of each predetermined point of the photographed image is calculated. When B (blue) is larger than R (red), the gain for R is increased. When B is smaller than R, the gain for R is lowered. When B is larger than G (green), the gain for G is increased. When B is smaller than G, the gain of G is lowered. The above steps (a)-(b) are repeated until each value reaches the target value. With this operation, setting values for the camera at the time of photographing is obtained. The same operation is also performed with the third light source and the first camera and with the fourth light source and the second camera.

### DESCRIPTION OF REFERENCE SINGS

100 ·······medicine dispensing device 110·······vial bottle feeding device 120·······label attachment device 130·······vial bottle carrying device 140·······.discharge port 150·······cassette arranged portion 160·······touch panel display 170·······optical scanner 180·······medicine supplying part 181·······RF tag reading device 182·······.unlocking device 190·······.control part 200.......medicine dispensing cassette 210·······main body 211·······RF tag 212·······hole 213·······right side inner wall 220·······first rotating body 230·······cylindrical portion 300·······first sub-member 310·······lid 311·······hinge 320·······.engaging portion 321·······protruding portion 322·······slit 323·······rib 400·······tray 410·······petri dish 411·······bottom portion 412·······side surface 413·······ring 414·······capsule placing member 415·······.transparent portion 416·······concave portion 417·······concave portion 420·······main body 421·······hole 422·······cylinder 500·······main member 600·······third sub-member 710·······first camera 720 ·······second camera 730·······first light source 731·······first ring light 740·······second light source 74·······second ring light 742·······lower support plate 743·······upper light source 750·······third light source 751·······first bar light 760·······fourth light source 761·······third bar light 762·······fourth bar light 800·······locking mechanism 810·······inclined portion 820·······shaft 830·······biasing member 831·······base end portion 832·······curved portion 833·······tip end portion 840·······engaging member 900·······medicine photographing device 910·······case 911·······lower case 912·······cutout portion 913·······upper case 914·······cover 915·······inclined portion 916·······slope 917·······cutout space 920·······placing portion 921·······tray support member 922·······hole 923·······engaging portion 930·······guide member 931·······slope 940·······upper internal space 950·······frame

## Claims

1. A medicine loading device for loading a medicine based on prescription information, comprising:
a main body;
at least one medicine dispensing cassette provided in the main body; and
medicine discriminating means for detecting a type of a medicine,
wherein an openable/closable lid and a locking mechanism for locking the lid are provided at the medicine dispensing cassette, and
wherein when the type of the medicine detected by the medicine discriminating means and a type of a medicine to be contained in the medicine dispensing cassette match each other, the locking mechanism is released.

2. The medicine loading device as claimed in claim 1, wherein the medicine discriminating means further has a medicine information obtaining part for reading cassette information provided at either one of the medicine dispensing cassette and the lid and medicine information provided on the medicine and an information processing part connected to the medicine information obtaining part, and
wherein the information processing part discriminates the type of the medicine based on information on the medicine obtained by the medicine information obtaining part.

3. The medicine loading device as claimed in claim 2, wherein the medicine information obtaining part includes a photographing device for obtaining an image in which the information on the medicine which is a discrimination target is indicated and at least one of a first reading device which can read a code in which the information on the medicine which is the discrimination target is indicated and a second reading device which can read a signal indicating the medicine information of the discrimination target.

4. The medicine loading device as claimed in claim 3, wherein the image in which the medicine which is the discrimination target is indicated is an image of an engraved mark or a print of the medicine, and
wherein the code in which the medicine which is the discrimination target is indicated is any one of a bar code, a two-dimensional code and a three-dimensional code indicating the medicine information on the medicine.

5. The medicine loading device as claimed in any one of claims 1 to 4, wherein the locking mechanism further has a trigger for allowing a lock to be released, and
wherein the trigger can further return the locking mechanism to a locked state after releasing the locking mechanism.

6. The medicine loading device as claimed in claim 5, wherein the trigger allows the cassette to be removed from the medicine loading device and the trigger is returned from a releasing state of the lock to an original state.

7. The medicine loading device as claimed in claim 5, wherein the trigger includes a receiving part for receiving an instruction for allowing the trigger to return from a releasing state of the lock to an original state from an outside after the cassette has completed a loading operation.

8. The medicine loading device as claimed in claim 5, wherein the trigger further has a timer part, and
wherein the trigger is returned from a releasing state of the lock to an original state when a predetermined time elapses after the locking mechanism has been released by the timer part.

9. The medicine loading device as claimed in any one of claims 1 to 8, wherein the medicine loading device further has a storage part which can store predetermined medicine information in advance and further store the information on the medicine detected by the medicine discriminating means, and
wherein the storage part stores an image of the medicine, a numerical value or a rank indicating a degree of possibility of correctness and an engraved mark or a print code of the medicine.

10. The medicine loading device as claimed in claim 9, wherein the medicine loading device further has a control part which creates template image data based on the stored information on the medicine and performs pattern matching for calculating a score serving as an index of a degree of matching between an image data of the engraved mark and/or a print of the medicine and the template image data to discriminate the medicine.

11. The medicine loading device as claimed in any one of claims 1 to 10, wherein the medicine loading device further has a display, and
wherein the display displays a result of comparing information on the medicine detected by the medicine discriminating means with information on the medicine stored in a storage part.

12. The medicine loading device as claimed in any one of claims 1 to 11, wherein the medicine loading device further has an alert part for alerting an operator when the type of the medicine detected by the medicine discriminating means and the type of the medicine to be contained in the medicine dispensing cassette do not match each other.

13. A medicine recovery method for recovering a medicine based on prescription information, comprising:
obtaining medicine information indicating a type of a medicine which is a discrimination target;
comparing the medicine information with cassette information indicating a type of a medicine to be contained in a medicine dispensing cassette;
determining whether the medicine information and the cassette information match each other; and
releasing a locking mechanism provided at the medicine dispensing cassette when the medicine information and the cassette information match each other.

14. A medicine loading device for preventing an improper medicine from being loaded into medicine holding means, comprising:
a main body;
at least one medicine holding means contained in the main body;
an opening and closing part attached to the medicine holding means;
a locking mechanism for locking the opening and closing part; and
discriminating means for discriminating a type of a medicine,
wherein when the type of the medicine discriminated by the discriminating means and a type of a medicine to be contained in the medicine holding means match each other, the locking mechanism is released.
